# EUROPEAN PATENT SPECIFICATION

(11) **EP 0 836 518 B1**
(45) Date of publication and mention of the grant of the patent: **06.12.2006**
(21) Application number: 96918299.7
(22) Date of filing: 07.06.1996
(51) Int. Cl.: A63B 23/18

(54) **A PORTABLE, PERSONAL BREATHING APPARATUS**
TRAGBARES UND PERSÖHNLICHES BEATMUNGSGERÄT
APPAREIL RESPIRATOIRE PERSONNEL PORTABLE

(30) Priority: 07.06.1995 US 478741
(43) Date of publication of application: 22.04.1998
(73) Proprietor: Everett D. Hougen Irrevocable Trust, Troy, MI 48084 (US)
(72) Inventor: Everett D. Hougen, Flint, MI 48503 (US)
(74) Representative: Shanks, Andrew
(86) International application number: PCT/US1996/009410
(87) International publication number: WO 1996/040376

(56) References cited:
- EP-A- 0 372 148
- WO-A-90/09203
- FR-A- 2 379 291
- US-A- 515 637
- US-A- 737 008
- US-A- 3 972 326
- US-A- 4 062 358
- US-A- 4 221 381
- US-A- 4 291 704
- US-A- 4 770 413
- US-A- 5 255 687

## Description

### BACKGROUND OF THE INVENTION

The present invention relates to a portable respiratory exercise apparatus providing resistance and intra-trachea bronchial percussion on inspiration and expiration to increase pulmonary efficiency, while improving cilial movement which assists mobilization of intrabronchial mucous or secretions within the lungs.

Research has shown that by practicing deep abdominal breathing, abdominal muscle pressure and temperature are raised, digestion and absorption of foods are improved and pulmonary efficiency is increased. In addition, taking deep breaths while performing little physical movement causes a superfluous amount of oxygen to be made available. Because the large muscular tissue is not consuming the oxygen an increased oxygen supply is made available for many other body systems, such as the brain and the heart.

Forcible and prolonged inspiration and expiration causes a greater expansion and collapse of the air vesicles (alveoli), especially those deep in the lung tissue. By providing resistance to inspiration and expiration, pulmonary muscles are strengthened and developed, thereby allowing a freer and greater exchange of oxygen and carbon dioxide. Persons suffering from lung ailments, healthy persons, and athletes can all improve their pulmonary efficiency through forcible and prolonged inspiration and expiration against resistance.

Some people are able to take only shallow breaths because they are suffering from lung ailments such as asthma, emphysema, chronic bronchitis, chronic obstructive pulmonary disease, or other ailments which reduce the oxygen/CO₂ exchange. Frequently, patients recovering from abdominal surgery experience pain during deep breathing and may therefore restrict their own breathing to shallow breaths. In both of the above situations, recovery is slowed because the patients suffer from reduced exchange of oxygen and carbon dioxide in the tissue. Further, the patients are at risk of developing atelectasis because their lungs are not being fully expanded. Atelectasis is a partial collapse of the lungs, possibly leading to necrosis of the lung alveoli. This exacerbates any ailments from which the patient may be suffering by causing poor oxygen exchange in the lungs and possibly resulting in pneumonia.

Patients with emphysema further suffer from mucous blockages in the lungs. Cilia, tiny hairlike structures in the lungs, become flattened down and clogged by mucous. Vibration of the air during inspiration or expiration can cause vibration of the lungs, lung passages (bronchi), and cilia of the patient. This vibration sometimes provides relief to the patient by bringing the cilia to an upright position and mobilizing the mucous, facilitating the expectoration thereof.

Known respiratory exercisers utilize a ball inside a large tube. A user exhales or inhales through a smaller attached tube, causing the ball to rise proportionally to the rate of airflow. However, these known respiratory exercisers only provide resistance to inspiration or expiration, but not both. Further, the large tube must be maintained in a vertical position in order for the respiratory exerciser to operate correctly. This is inconvenient for persons suffering from lung ailments who may be confined to bed and for athletes who wish to restrict respiratory volume flow during exercise. Still further, this respiratory exerciser does not provide a percussive effect on the user; i.e., a vibration of the air on inspiration or expiration.

Another known respiratory exerciser provides a mask which allows air to be inhaled freely and provides resistance against the expiration of air. The masks do not provide resistance to inspiration and do not provide vibration. Further, the masks are too large to be conveniently portable.

Another respiratory exerciser provides a vibration effect upon expiration. A patient exhales into a tube connected to a conical element loosely supporting a ball. When a patient exhales through the tube, the ball is displaced from the conical element causing an oscillatory movement of the ball, thereby generating a variable pressure opposing the expiration. There are several disadvantages to this device. It does not provide vibration of air during inspiration. It is inconvenient for some patients because it must be maintained at a horizontal position during use. Further, the device provides only varying oscillations in air pressure, rather than a sharp percussion of the air by rapid bursts of air pressure from complete opening and closure of the air passages.

Another respira-tory exerciser provides a vibration effect upon either inspiration or expiration by using a pair of adjacent air passageways each containing a reed. Each passageway contains a valve utilizing a coil spring to allow either inspiration or expiration. The compression of each spring can be adjusted to vary the resistance to inspiration and expiration independently. As the patient inhales through one passageway and exhales through the other, air flowing past each reed causes each reed to rapidly vibrate, causing a vibration effect on the lungs of the patient. However, adjustment of the coil spring compression during inspiration and expiration is not convenient. Further, vibration of the air is not as effective as would be a sharp percussion of the air by rapid, complete opening and closure of the air passages.

FR-A-2 379 291 discloses a lung exercising device comprising an outer member, an inner member reciprocally and rotatably mounted with the outer member, a mouthpiece in communication with the inner member, at least are opening in the under and outer members adapted to be in selective communication to device an air passage extending from the mouth piece through the inner member and out of the outer member.

### SUMMARY OF THE INVENTION

The present invention, as claimed in claim 1, provides a respiratory exercise apparatus which is portable, non-positional, and provides percussion and resistance during inspiration and expiration and pulsing during expiration. The user can select whether to exercise through resistance, percussion, or pulsing. The breathing apparatus has a main body and a movable inner member, which in the preferred embodiment is an inner cylinder. Preferably, both the main body and the inner cylinder have holes and slots for resistance, pulse, and percussion exercises. By rotating the inner cylinder and locking it in place, the desired method of exercise can be selected and performed.

Resistance exercises can be performed by aligning holes in the main body and the inner cylinder. Alignment and locking are accomplished through use of grooves and a locking pin, screw, boss, etc. In the preferred embodiment, the inner cylinder has a plurality of grooves with the grooves being preferably oblong and adapted to receive the locking pin to restrict movement. The oblong grooves allow limited reciprocal movement of the inner cylinder with respect to the main body and prevent rotational movement. This restricted movement allows mating holes to move with respect to one another and automatically vary the resistance as a user inhales and exhales. Alternatively, the inner cylinder can be manually reciprocated to vary the resistance or held in place to maintain a predetermined resistance. In the preferred embodiment, there are several paired holes of varied diameter to give varied resistance. These holes are selected by rotating the inner cylinder, aligning a desired set of holes and locking the inner cylinder in place. In the preferred embodiment, there is a flange that has indicator holes that facilitate easy alignment.

A further resistance exercise can be performed by holding the breathing apparatus generally vertically with the inner cylinder pointing up and exhaling. This will raise the inner cylinder. When the lungs are empty, the inner cylinder automatically drops back into the main body. The user then tries to raise the inner cylinder again by exhaling any remaining air in the lungs. The device is then held with the inner cylinder down, which causes the inner cylinder to automatically drop down. The user then inhales to pull the inner cylinder back into the main body until the lungs are full, which then causes the inner cylinder to drop back down. The exercise is completed by further inhaling to try to raise the inner cylinder.

Percussion exercises can be performed by locking the locking member in a second set of grooves which will align slots in the main body and the inner cylinder. In the preferred embodiment, this groove is formed about the circumference of the inner cylinder to allow limited rotational movement of the inner cylinder with respect to the main body. The user can percuss the lungs by rapidly rotating the inner cylinder while the user inhales and exhales into the main body. Rotation of the inner cylinder moves the slots in the inner cylinder and main body with respect to one another allowing quick bursts of air to enter and exit the users lungs. This provides a strong percussion effect which expands the air vesicles deep in the lungs and loosens mucous blockages in the lungs. Because the present invention provides rapid intermittent complete closure of the airflow in and out of the user's lungs during percussion, a more effective percussion effect is obtained than by merely vibrating the air pressure.

Pulse exercises are performed by aligning the locking device with a longitudinally extending groove. This allows the inner cylinder to reciprocate sufficiently to expose the inner slots. To perform the exercise, the device is held upright, and the user exhales, which causes the inner cylinder to raise, expose the slots, and drop repeatedly. In the preferred embodiment, the inner cylinder is weighted to facilitate the reciprocating action.

In the disclosed embodiments, the respiratory exercise apparatus includes a generally cylindrical main body having at least one main aperture. Although disclosed as cylindrical, in some embodiments, the main body can be non-cylindrical. An inner cylinder is disposed within the main body and can rotate and reciprocate within the main body.

It will be apparent to one of ordinary skill that other embodiments could be used to obtain similar results and objectives and still be within the scope of the invention. With reference to the following Brief Description of the Drawings and disclosure, the invention will be described.

### BRIEF DESCRIPTION OF THE DRAWINGS

The above, as well as other advantages of the present invention will become readily apparent to those skilled in the art from the following detailed description of a preferred embodiment when considered in the light of the accompanying drawings in which:
Fig. 1 is a perspective view of a respiratory exercise apparatus in accordance with the present invention;
Fig. 2 is an end view of the respiratory exercise apparatus of the present invention as viewed from the mouthpiece.
Fig. 3 is a partially cut away perspective view of a further embodiment of the present invention.
Fig. 4 is an exploded perspective view of a further embodiment of the present invention.
Fig. 5 is a side view of the breathing device of figure 4 in the vertical position.
Fig. 6 is a side view of the breathing device of figure 5 rotated 180°.
Fig. 7 is a side view of the breathing device of figure 4 in the horizontal position.
Figs. 8 through 11 illustrate various flow meters.
Fig. 12 is a partial cut away view of the breathing device base.
Figs. 13 and 14 illustrate modified breathing devices having slanted bases.

### DETAILED DESCRIPTION OF THE INVENTION

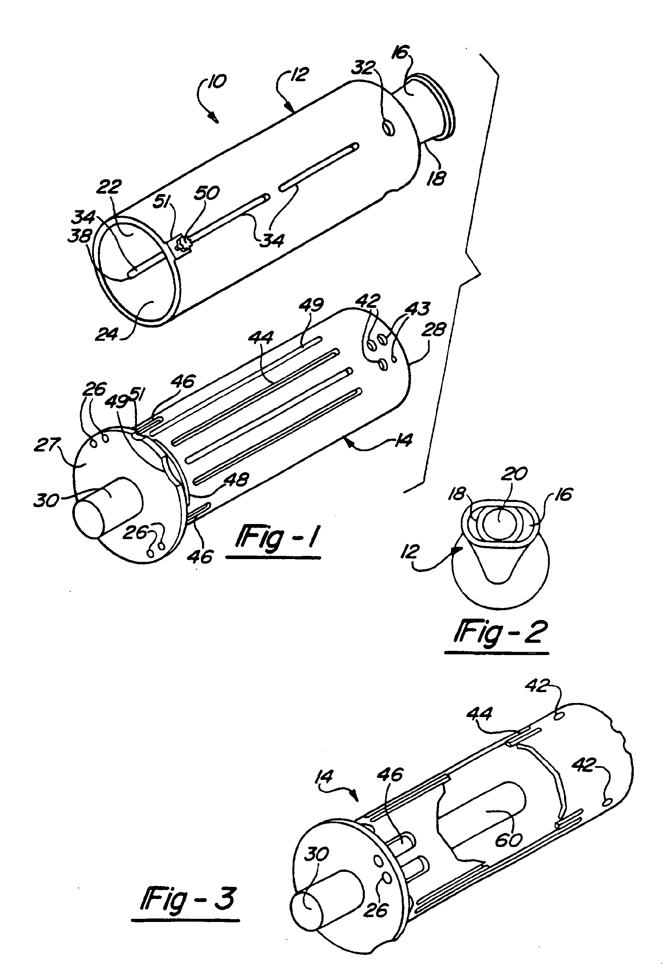

With reference to Figure 1, the breathing apparatus of the present invention is generally shown at 10. The breathing apparatus includes a main body 12 and an inner member 14, which is preferably a cylindrical tube. In the illustrated embodiment, the main body 12 is also illustrated as a cylindrical tube, but it should be understood that the shape of the main body 12 could be any desired shape. Further, if the breathing device does not include the percussion exercise, the inner member 14 could be any desired shape which would allow reciprocal movement with respect to the main body 12.

The main body 12 has a mouthpiece 16 attached to a flange 18. The flange 18 is preferably angled at about 45° and includes a bore 20 that communicates with the interior 22 of the main body 12. In the preferred embodiment, the mouthpiece 16 is sufficiently flexible to be stretched over flange 18 to change the angle of the mouthpiece from 45° to generally in line with the main body 12. The opposite end 24 of the main body 12 is open for receipt of the inner cylinder 14. The inner cylinder 14 has a closed end 26 and an open end 28 with the open end 28 being inserted into main body 12. A knob 30 extends from the end 26 so that the inner cylinder 14 can be manually rotated or reciprocated within the main body 12. In the preferred embodiment, the main body is 90.602 mm (3.567") long, which includes the mouthpiece 16, and the inner cylinder is 93.218 mm (3.67") if the knob 30 is included. The main body and inner cylinder are made of polycarbonate or other moldable plastic, such as polyethylene.

The breathing apparatus 10 is adapted for exercising a users lungs through inspiration and expiration against resistance pulsing and percussion. The apparatus 10 has two sets of openings that can be aligned to provide resistance, pulsing or percussion exercises. By rotating the inner cylinder 14 with respect to the main body 12, the different sets of openings can be aligned and locked in position to allow for a selected exercise.

In the illustrated embodiment, the main body 12 has a hole 32 and a pair of elongated openings or slots 34 and 38 which are about 180° apart. The inner cylinder 14 has a plurality of paired holes 42 and 43 and elongated openings or slots 44 which extend through the inner cylinder 14. These holes 42 and 43 and slots 44 are positioned to align with the holes 32 and elongated slots 34 and 38 respectively as the inner cylinder 14 is rotated with respect to the main body 12. When the holes 42 and 43 are aligned with the hole 32, resistance exercises can be performed. A set of grooves 46 which correspond to the holes 42 are provided in the inner cylinder for holding the inner cylinder 14 with respect to the main body 12. Grooves 46 are adapted to receive a locking member 50 to lock the cylinder in position once an appropriate hole 42 is aligned with hole 32.

When percussive exercises are desired, the slots 34, 38, and 44 are aligned. A different set of grooves 48 are provided, which correspond to the slots 44. Grooves 48 extend circumferentially about the cylinder to restrict movement of the cylinder 14 and main body 12 so that only the slots are aligned for percussive exercises. As will be further described below, the grooves 48 permit limited rotational movement of cylinder 14 with respect to main body 12.

When pulsing is desired, the locking member is inserted into a longitudinally extending groove 49, which extends along the inner cylinder. This groove allows the inner cylinder 14 to reciprocate with respect to the main body 12.

In the disclosed embodiment, the locking member 50 is a small bolt that is threaded into a threaded bore. A raised portion 51 prevents the member 50 from being threaded too far into the grooves. The bolt can be screwed into the groove as desired. It should be understood that other types of locking members could be used, such as a spring loaded detent, a fixed detent, etc.

The grooves 46, which correspond to the holes 32 and 42, are preferably oblong and have their longitudinal axis extending parallel to the longitudinal axis of the inner cylinder 14. Preferably, the locking member 50 has a diameter that is less than the length and width of the groove 46 so that the inner cylinder can reciprocate slightly within the main body 12. Due to the movement of the inner cylinder 14 with respect to the main body 12, the resistance against exhaling can be increased; i.e., the ability to exhale is further restricted, due to the shifting from the hole 43 to the hole 43. When the user inhales, the inner cylinder 14 is pulled back into the main body 12 to shift from hole 43 to hole 42, reducing resistance upon inhalation.

It should be appreciated that by locating the holes 42 and 43 differently, the reverse could be accomplished and greater resistance could be provided when inhaling as opposed to exhaling. Still further, the user can adjust the resistance to inhaling and exhaling by manually sliding the inner cylinder 14 with respect to the main body 12. Again, since the inner holes are sliding with respect to the outer hole 32 in main body 12, the resistance is changed.

In the preferred embodiment, the hole sets 42 and 43 have different diameters to provide varied resistance, depending upon whether the user is exhaling or inhaling. In the preferred embodiment, the diameters of the holes 42 are 4.75 mm (.187"), 3.96 mm (.156"), 3.18 mm (.125") and 2.36 mm (.093"); the diameters of holes 43 are 2.77 mm (.109"), 2.36 mm (.093"); 1.98 mm (.078"), and 1.57 mm (.062"); and the diameter of the hole 32 is 4.75 mm (.187"). By rotating the cylinder 14, various resistances can be selected and then automatically varied. Further, in the preferred embodiment, there are grooves 46 corresponding to each hole set 42 and 43. Alignment holes 26 are provided in flange 27. These holes 26 correspond in size to their corresponding holes. By locating the holes with the locking member 50, the desired holes 42 and 43 are aligned. Alternatively, by aligning the desired diameter hole 42 with the hole 32, the locking member 50 can be inserted into the adjacent groove. In the disclosed embodiment, there are four (4) sets of holes 42 and 43, four (4) grooves 46 and one hole 32. The holes 42 and 43 are separated into two groups with each set about 180° apart and the hole sets in each group are about 30° apart. The grooves 46 are also separated into two sets with each set about 180° apart and the grooves in each set being about 30° apart. The hole sets 42 and 43 and the grooves 46 are about 90° apart.

With the mouthpiece 16 angled at approximately 45°, the air that escapes from hole 32 blows against the user's face. This provides the user with an indication of the effort being expended and can function as an incentive to continue exhaling effectively.

Percussion is dependent upon grooves 48 which extend between the grooves 46 and correspond to the alignment of the inner slots 44 and the outer slots 34. In the preferred embodiment, each groove 48 extends circumferentially about the inner cylinder through an arc which is greater than 90°. When the locking member 50 is positioned in one of the grooves 48, the inner cylinder 14 can be rotated with respect to the main body 12 through an arc defined by the groove; i.e., approximately 90°. When the inner cylinder 14 is rotated, the slots 44 sweep by the slots 34 to rapidly open and close the slots and rapidly block and open the ingress and egress of air into and out of the breathing apparatus 10. This rapid action causes a percussive result in the users lungs. It should be appreciated by those of ordinary skill in the art that this invention permits percussive exercising of the lungs on inhalation and exhalation.

In the disclosed embodiment, the grooves 48 are positioned apart by about 180°. Each groove is adjacent the end of the slots 44 and extends circumferentially beyond the slots a slight distance to enhance the percussive effect the user receives upon use. Preferably, there are two sets of slots 44 and two grooves 48. Only one set of slots 44 and grooves 48 are shown. Additionally, there are preferably two sets of slots 34 on opposed sides of main body 12. The groove 48 opposite the aligned slot 44 is the groove that receives the locking member 50. It should be appreciated that the groove 48 adjacent the slot 44 could be used if the locking member or the slot 34 were repositioned during manufacture.

In the preferred embodiment, the slots 34 and 44 are long and narrow to give increased percussive effect to the user. The disclosed slots 34 and 44 are approximately 50.8 mm (two (2)) inches long and 1.57 mm (.062) inches wide. For strength, each of the slots 34 and 44 can be formed by two smaller slots positioned end to end. Applicant has found that long narrow slots provide a more effective percussion of the lungs because greater volumes of air can rapidly pass through the slots, and the slots can be closed rapidly, resulting in bursts of air into the lungs. Because of the burst of air, a humming noise is produced, which can also act as an incentive for increased usage. A notch 49 is provided to permit easier alignment of the slots 34 and 44. By aligning notch 49 with locking member 50, the slots are automatically aligned.

To provide pulsing exercise, the groove 49 is aligned with locking member 50. A smaller notch 51 is provided to facilitate proper alignment. By aligning notch 51 with locking member 50, groove 49 is directly beneath locking member 50 and can then receive locking member 50. Groove 49 allows the inner cylinder to reciprocate with respect to the main body 12 as the user exhales. With the unit pointed up, the user exhales and the inner cylinder is raised to expose slots 44. When slots 44 are exposed, the inner cylinder 14 drops down momentarily until pressure from the exhaled air increases in the cylinder 14 to raise it again. This repeated raising and dropping causes a pulsing effect on the lungs.

To use the breathing device, the inner cylinder 14 is rotated with respect to the main body 12 to align either the holes 32 and 42 or the slots 34 and 44 or the groove 49. If resistance exercising is desired, then the holes are aligned. If percussion is desired, then the slots are aligned. If pulsing is desired, then groove 49 is aligned. Once aligned, the locking member 50 is threaded into the adjacent groove. Once the desired exercise is selected, the user inhales and exhales into the mouthpiece 16. If resistive exercising is selected, the inner cylinder 14 reciprocates within the main body 12 to automatically adjust the resistance, or the user can manually control the relative resistance by controlling the amount of reciprocal movement of the inner cylinder 14. If percussion is selected, the inner cylinder 14 is manually rotated by the user as the user inhales and exhales. Knob 30 facilitates rotation of inner cylinder 14 with respect to main body 12. If pulsing is desired, the user uses groove 49.

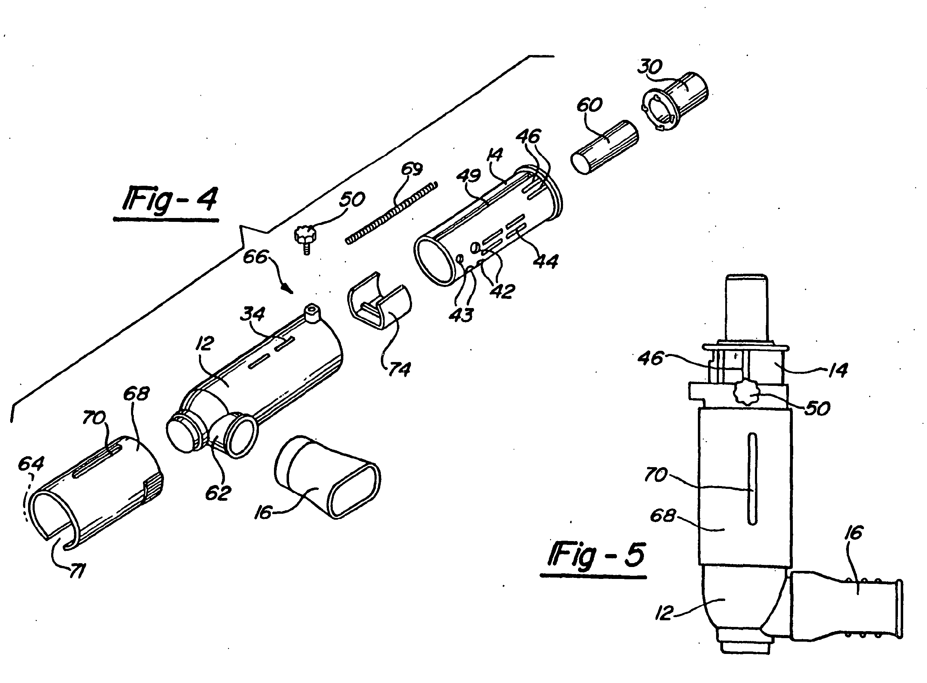

With reference to figure 4, a further embodiment of the present invention is shown. In this embodiment, a weight 60 is positioned in the inner cylinder 14. The weight is illustrated as a rod, but it could be a ball, a disk attached to the flange 27, a spring, etc. The weight 60 facilitates the reciprocal movement of the inner cylinder 14 with respect to the main body 12. The weight 60 forces cylinder 14 out of the main body 12 to further restrict inhalation and exhalation or with pulsing to force cylinder 14 back into the main body 12.

With reference to figure 4, a further embodiment of the present invention is illustrated generally at 66. In this embodiment, there is a main body 12, an inner member 14 and a mouthpiece 16 as in the previous embodiment illustrated in figures 1 through 3. The main body is about 101.6 mm (4 inches) long and 31.25 mm (1.25 inches) in diameter; the inner member 14 is sized to fit into the main body 12. However, breathing device 66 provides for additional exercises as well as the exercises that can performed with the previously described breathing device 10. The inner member 14 has been modified and covers are provided to allow for the additional exercises. Additionally, the mouthpiece 16 is mounted to a short nipple 62 that extends out of the main body 12 at about a 90° angle to the longitudinal centerline 64 of main body 12. The nipple 62 is a 22.23 mm (7/8 inch) diameter protrusion with a 19.05 mm (3/4 inch) inside diameter. The orientation of mouthpiece 16 at a 90° angle allows the user of the breathing apparatus to twist the mouthpiece 16 on nipple 62 to perform various exercises which will be described more fully below and to vary the resistance when performing these exercises.

As with the previous embodiment, inner member 14 of device 66 is inserted into the end of main body 12 so that it can be reciprocated and rotated with respect to main body 12. A knob 30 extends from the end of inner member 14 to rotate and reciprocate the inner member 14.

In addition to the previously described exercises of resistance, pulsing or bouncing and percussion; breathing device 66 also permits a pumping mode and a slow sustained maximal expiration and inspiration mode. It also includes a spring 69 to enhance the movement of the inner member 14 in the pulsing or bouncing mode. To simplify the description, the same reference numbers will be used to identify the same features found in the previous embodiment. Unless otherwise indicated, the same functions are associated with the same elements. As before, resistance exercises are provided by aligning the thumb screw with grooves 46, of which there are three in this device, with one being out of view. With screw 50 in groove 46 hole sets 42 and 43 are aligned with opening 32 (which is also not illustrated in this figure but is illustrated in figure 1). In the preferred, there are three sets of holes 42 and 43 which are sized with a 3:1 ratio in each pair set. The large hole is used for inhaling and the small hole for exhaling. Furthermore, each pair of holes 42 and 43 are of different diameter. In the preferred embodiment, the grooves 46, or which there are three, are approximately 15.88 mm (5/8 inches) in length. The location grooves 46 align corresponding hole sets 42 and 43 with the exhaust opening 32. In the preferred embodiment, exhaust hole 32 is about 6.35 mm (1/4 inch) in diameter. Percussion exercises are performed by aligning slots 44 with slots 34. In the preferred embodiment, there are two groups of six percussion slots 44 located 180° apart from one another. The slots are preferably 1.59 mm (1/16 inch) wide by 12.7 mm (1/2 inch) long. The slots 34 are in two pairs, 180° apart and are 1.59 mm (1/16 inch) wide by 12.7 mm (1/2 inch) long. A groove 48 is provided in the inner member 14 to accept the thumbscrew 50. Groove 48 is also not shown in this embodiment, since it is out of view; however, it is shown in figure 1. When pulsing is desired, the thumbscrew 50 is inserted into groove 49. As before, the inner cylinder 14 reciprocates with respect to the main body 12. As illustrated, a spring 69 is mounted in the groove 49. The thumbscrew 50 when inserted into groove 49 engages one end of the spring and acts against the spring to bias movement of the inner member 14. In this operation mode, the slots 34 and opening 32 in the main body are covered to prevent air from escaping through them. The slots 34 and opening 32 are preferably covered by the inner member 14. However, other methods of covering the openings could be used, such as for example a flexible sleeve 68 that can be snapped over the body member 12. This sleeve 68 can be rotated to close or open slots 34 and opening 32 by aligning the slot 70 and opening 71 of the sleeve 68 over the slots 44 and opening 32. Still further, the main body could be made without the opening 32 and lots 34.

In the pulse mode, the user exhales and the inner member 14 slides away from main body 12 and compresses the spring 69. The spring provides resistance to the outward movement of the inner member 14. When the openings 44 are exposed from the main body 12, air exhaled by the user escapes through the exposed openings 44 and the inner member 14 snaps back into the main body 12 due to the bias of the spring 69 and the weight 60. In the preferred embodiment, the weight is about 12.7 mm (1//2 inch) in diameter by 38.1 mm (1 1/2 inches) in length. As the user continues to exhale, movement of the inner member 14 in and out of body 12 occurs very rapidly providing a bounce or pulse action. The snap back action rapidly closes and opens the air passages in the device 66 interrupting the flow of air creating resistance. This pulsing or bouncing action causes a very pronounced change in pressure in the lungs loosening mucous and strengthening the lungs. Preferably in the pulse mode, the user inhales deeply and then exhales into the mouthpiece 16 completely until all bouncing movement stops. The user then inhales deeply and repeats the exercise.

Slow sustained maximal expiration and inhalation can also be performed with this invention. With reference to figure 5, by rotating the inner cylinder 14 to align the thumbscrew 50 with one of the grooves 46, the inner member 14 can reciprocate out of main body 12 about 12.7 mm (1/2 inch). As illustrated in figure 4, the slots 44 do not extend into the area adjacent the longitudinal extent of grooves 46. In this mode, the only air passage which is open is the air passage created by aligning the openings 42 and 43 with opening 32. If desired, this air passage can be closed by holding a finger over the air passage or by rotating the cover 68 over the passage. If the passage is closed, air can still escape from the device 66, but it is greatly restricted.

In the preferred embodiment, the air passage created by openings 42, 43 and 32 remains open. By using openings 42, 43 and 32, the resistance automatically changes so that the proper resistance is selected to completely fill or empty the lungs. By holding the main body 12 upright in a vertical position, the user can exhale into the mouthpiece and raise the inner member 14. As long as there is sufficient air in the user's lungs to overcome the weight of the inner member 14, in the gravitational pull, the inner member 14 will remain raised. As soon as all sufficient air is expelled from the user's lungs, the inner member will drop back into the body member 16. The resistance can be varied by turning the mouthpiece 16 at a different angle on the nipple 62 and by selecting one of the other grooves 46. By angling the mouthpiece 16, the main body 12 and inner member 14 are angled with respect to the vertical which reduces resistance.

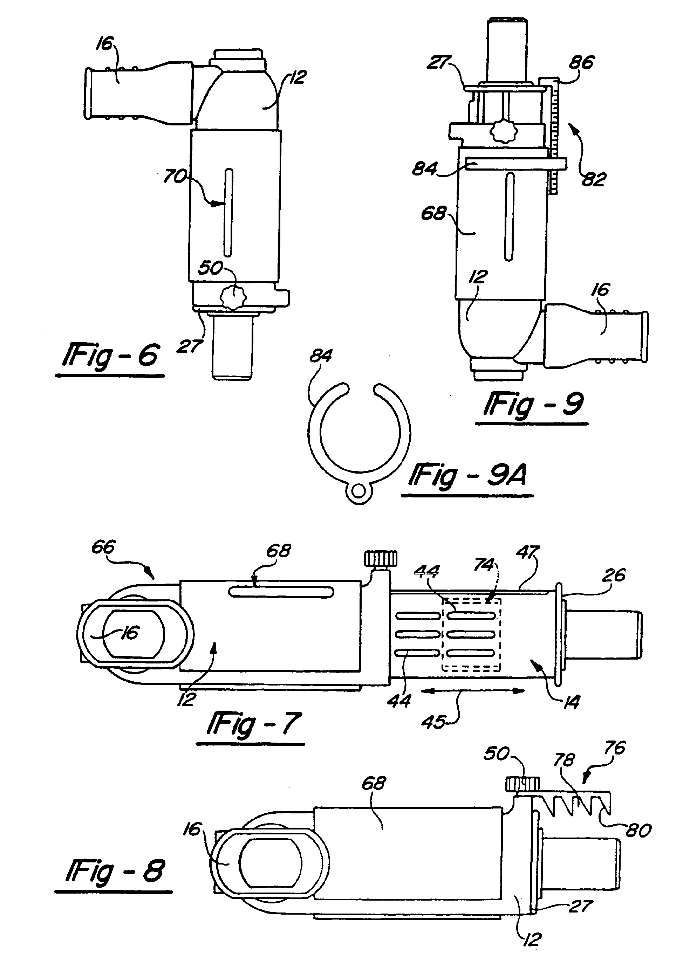

By rotating the breathing device 66 180° the inner member 14 points downwardly, see figure 6. The user can then inhale completely so that the inner cylinder 14 snaps up into the main body 12. When the lungs are completely filled, the main body 12 will drop down due to the weight 60 pulling the inner member 14 down. Again, by angling the mouthpiece 16, the resistance can be varied. In this mode the user experiences slow sustained maximal inspiration.

The other exercise that can be used is the pump mode. In this mode, illustrated in figure 7, an internal cover 74 is used to partially cover slots 44 which are adjacent closed end 26 and cover 68 is used to cover slot 34 and opening 32. The breathing device 66 is turned so that main body 12 is generally horizontal. The thumbscrew 50 is inserted into groove 49 or an additional groove 47 could be used, as illustrated in figure 7. The user can then reciprocate the inner member 14 with respect to the body member 12 as the user inhales and exhales, see arrow 45 in figure 7. As should be appreciated, the mouthpiece 16 is twisted on the nipple 62. The user inhales completely while quickly moving the inner member 14 in and out of the main body 12. The same is done while the user exhales completely. This mode very effectively maximizes air pressure changes created by the pumping action. This mode provides an effect similar to that achieved in the percussion mode.

With reference to figures 8 through 11, flow meters or progress meters are illustrated to indicate the amount of air flow and progress a user is making using the breathing devices of the present invention. In figure 8, the flow meter 76 attaches onto the end of the main body 12 at the thumbscrew 50. The thumbscrew can be used to hold the meter 76 in position. In the preferred embodiment, flowmeter 76 flexes slightly to allow the inner cylinder 14 to move along the notches 78. The edge of the meter 76 has a plurality of notches 78 with cam surfaces 80. The inner member 14 engages the cam surfaces 80 as it slides out of the main body 12 and gently forces meter 76 back to allow the flange 27 to pass. When the inner member 14 stops sliding out of the inner member 14 it is held by a one of the notches 78. The notches 78 are calibrated to indicate the air flow through the breathing device 66. In the preferred embodiment, the inner member would be rotated to a groove, such as groove 72 and the slots 34 and opening 32 would be opened by cover 68 to insure that inner member 14 is not blown out of main body 12. The calibration would take into account the flow lost through the open passages.

In figure 9, a further embodiment of a flow meter is shown generally at 82. In this embodiment, a guide 84, see figure 9a, is snapped onto the main body 12 with an indicator rod 86 engaging the flange 27. The rod 86 is L-shaped to hook onto flange 27 and includes graduations to indicate the amount of flow and progress. When the user exhales, the rod 86 is moved and remains as the inner cylinder 14 returns.

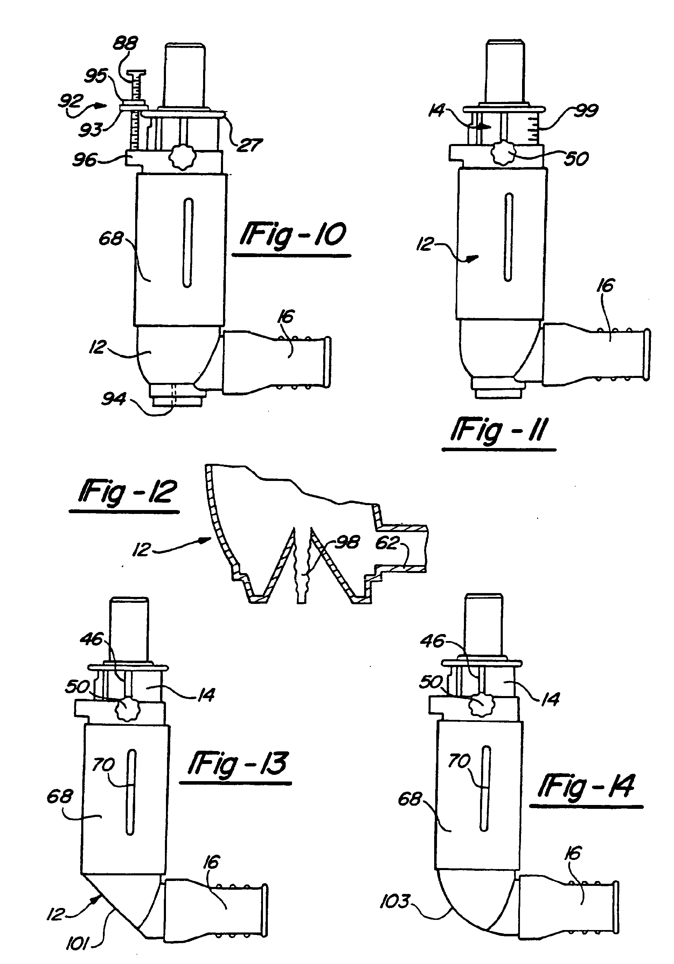

In figure 10, a still further embodiment is illustrated. In this embodiment, a removable rod 88 is rigidly mounted into a receptacle 90 formed in the main body 12. Additionally, the rod 88 could be attached to the thumbscrew 50. An indicator 92 is reciprocally mounted about the rod 88. The disclosed indicator 92 is a nylon washer 93 and felt washer 95 which can slide along rod 88. The washers engage the flange 27 and are moved along the rod 88. The felt washer remains to indicate the air flow and progress of the user. In this embodiment, the rod 88 can be inserted into an opening 94 formed in the base of main body 12.

In figure 11, another embodiment of a flow meter is illustrated. In this embodiment, the inner member 14 has graduated markings 99. As the user exhales, the mark on the inner member 14 will rise to indicate the air flow and progress. The user will be able to view the height reached and determine flow and progress.

In figure 12, an oxygen connector 98 is illustrated. In the disclosed embodiment the connector 98 is inset in main body 12; however, it could be externally mounted. Figures 13 and 14 illustrate further embodiments which have a slanted base 101 in figure 13 and 103 in figure 14. The slanted base facilitates air flow from mouthpiece 16 through the members 12 and 14.

In accordance with the provisions of the patent statutes, the present invention has been described in what is considered to represent its preferred embodiment. However, it should be noted that the invention can be practiced otherwise than as specifically illustrated and described without departing from the scope of the claims.

## Claims

1. A lung exercising device comprising:
a main body;
an inner member reciprocally and rotatably mounted within the main body;
a mouthpiece mounted to the main body and in communication with the inner member;
at least one opening in the inner member and at least one opening in the main body adapted to be in selective communication to define an air passage extending from the mouth piece through the inner member and out of the main body, the air passage being selectively variable upon rotational or reciprocal movement of the inner member with respect to the main body.
whereby the lung exercising device can provide various exercises for the lungs by manipulation of the inner member with respect to the main body.

2. The lung exercising device of claim 1, wherein the openings in the inner member include at least two apertures of different diameter spaced longitudinally along the inner member and adapted to communicate with the at least one opening in the main body as the inner member reciprocates within the main body;
whereby the user of the device can exhale and inhale into the mouthpiece to reciprocate the inner member with respect to the main body and automatically vary the resistance between inhalation and exhalation.

3. The lung exercising device of claim 2, wherein the openings in the inner member include a plurality of apertures separated into pairs to provide further selective resistance to inhaling and exhaling.

4. The lung exercising device of claim 1, wherein the openings in the inner member and main body include slots which can be selectively aligned to define the air passage.

5. The lung exercising device of claim 1, wherein the openings in the inner and main body are slots that can be selectively aligned by rotating the inner member with respect to the main body and thereby open and close the air passage as the slots pass over one another;
thereby creating a percussive action upon the user's lungs as the user inhales and exhales into the mouthpiece.

6. The lung exercising device of claim 1, wherein the openings in the inner member include at least two apertures of different diameter spaced longitudinally along the inner member which are adapted to communicate with the at least one opening in the main body as the inner member reciprocates within the main body; and
the openings in the inner member and main body include slots that can be selectively aligned by rotating the inner member with respect to the main body and thereby open and close the air passage as the slots pass over one another;
a control selector for selecting between the apertures and slots;
thereby creating a percussive action upon the user's lungs as the user inhales and exhales into the mouthpiece if the slots are selected or if the apertures are selected the user can reciprocate the inner member with respect to the main body and automatically vary the resistance between inhalation and exhalation.

7. The lung exercising device of claim 6, wherein the control selector includes a pin and grooves, the grooves being associated with either the slots or the apertures to provide the desired exercise.

8. The lung exercising device of claim 1, wherein the inner member is adapted to be reciprocated with respect to the main body by the user exhaling into the mouthpiece and raising the inner member, the openings being initially covered by the main body but being exposed as the inner member is raised with respect to the main body resulting in the inner member dropping back into the main body to be immediately raised with respect to the main body as the user continues to exhale;
whereby the user receives a rapid pulsing effect upon the lungs.

9. The breathing apparatus of claim 1, wherein said inner member and said main body have a plurality of grooves divided into at least first and second groups of grooves and a mating stop member, one of said groups of grooves having grooves which extend circumferentially with respect to said inner member and main body permitting rotational movement of said inner member with respect to said main body, said stop member mating with said grooves to control the extent of rotational movement, said rotational movement permitting percussive exercising;
the outer group of grooves having grooves which extend longitudinally with respect to said inner member and main body permitting reciprocal movement of said inner member with respect to said main body, said stop member mating with said other group of grooves to control the extent of reciprocal movement.

10. The lung exercising device of claim 1, further including a progress measuring device.

11. The lung exercising device of claim 1, further including a spring biasing the inner member with respect to the main body to resist the inner member from being moved with respect to the main body and to assist the return of the inner member with respect to the main body.

## Patentansprüche

1. Lungenübungsvorrichtung, welche umfasst:
einen Hauptkörper;
ein inneres Element, das auf eine hin- und herbewegbare sowie drehbare Art und Weise innerhalb des Hauptkörpers montiert ist;
ein Mundstück, das an dem Hauptkörper montiert ist und das mit dem inneren Element in Verbindung steht; und
mindestens eine Öffnung in dem inneren Element und mindestens eine Öffnung in dem Hauptkörper, welche so ausgelegt sind, dass sie in einer selektiven Verbindung stehen, um einen Luftdurchgang zu definieren, welcher sich von dem Mundstück durch das innere Element hindurch und aus dem Hauptkörper heraus erstreckt, wobei der Luftdurchgang selektiv variabel ist aufgrund einer Dreh- oder einer Hin- und Herbewegung des inneren Elements in Bezug auf den Hauptkörper;
wobei die Lungenübungsvorrichtung aufgrund eines Bedienens des inneren Elementes in Bezug auf den Hauptkörper verschiedene Übungen für die Lungen liefern kann.

2. Lungenübungsvorrichtung gemäß Anspruch 1, bei welcher die Öffnungen in dem inneren Element mindestens zwei Aperturen mit verschiedenen Durchmessern aufweisen, welche in der Längsrichtung entlang des inneren Elementes mit Abständen voneinander angeordnet sind und welche so angepasst sind, dass sie mit der mindestens einen Öffnung in dem Hauptkörper kommunizieren, wenn das innere Element sich innerhalb des Hauptkörpers bei der Hin- und Herbewegung befindet;
wobei der Benutzer der Vorrichtung in das Mundstück ausatmen und einatmen kann, um das innere Element in Bezug auf den Hauptkörper in eine Hin- und Herzubewegung zu versetzen und um den Widerstand zwischen der Einatmung und der Ausatmung automatisch zu verändern.

3. Lungenübungsvorrichtung gemäß Anspruch 2, bei welcher die Öffnungen in dem inneren Element eine Vielzahl von Aperturenn einschließen, die in Paare getrennt sind, um einen weiteren selektiven Widerstand gegenüber dem Einatmen und dem Ausatmen zu liefern.

4. Lungenübungsvorrichtung gemäß Anspruch 1, bei welcher die Öffnungen in dem inneren Element und in dem Hauptkörper Schlitze einschließen, welche selektiv ausgerichtet werden können, um den Luftdurchgang zu definieren.

5. Lungenübungsvorrichtung gemäß Anspruch 1, bei welcher die Öffnungen in dem inneren Element sowie in dem Hauptkörper aus Schlitzen bestehen, welche durch ein Drehen des inneren Elementes in Bezug auf den Hauptkörper selektiv ausgerichtet werden können und dass **dadurch** der Luftdurchgang geöffnet und geschlossen wird, wenn die Schlitze die einen über die anderen gleiten;
wodurch eine perkussionsartige Wirkung auf die Lungen des Benutzers ausgeübt wird, wenn der Benutzer in das Mundstück einatmet und ausatmet.

6. Lungenübungsvorrichtung gemäß Anspruch 1, bei welcher die Öffnungen in dem inneren Element mindestens zwei Aperturen mit verschiedenen Durchmessern aufweisen, welche in der Längsrichtung entlang des inneren Elementes mit Abständen voneinander angeordnet sind und welche so angepasst sind, dass sie mit der mindestens einen Öffnung in dem Hauptkörper kommunizieren, wenn das innere Element sich innerhalb des Hauptkörpers bei der Hin- und Herbewegung befindet; und
die Öffnungen in dem inneren Element und in dem Hauptkörper Schlitze einschließen, welche selektiv ausgerichtet werden können, durch ein Rotieren des inneren Elementes in Bezug auf den Hauptkörper und dass **dadurch** der Luftdurchgang geöffnet und geschlossen wird, wenn die Schlitze die einen über die anderen gleiten;
eine Steuerauswahlvorrichtung zum Auswählen zwischen den Aperturenn und den Schlitzen dient;
wodurch eine perkussionsartige Wirkung auf die Lungen des Benutzers erzeugt wird, wenn der Benutzer in das Mundstück einatmet und ausatmet, wenn die Schlitze ausgewählt sind, oder wenn die Aperturen ausgewählt sind, wobei der Benutzer das innere Element einer Hin- und Herbewegung in Bezug auf den Hauptkörper unterziehen kann und den Widerstand zwischen der Einatmung und der Ausatmung automatisch verändern kann.

7. Lungenübungsvorrichtung gemäß Anspruch 6, bei welcher die Steuerauswahlvorrichtung einen Stift und Einschnitte einschließt, wobei die Einschnitte entweder mit den Schlitzen oder mit den Aperturenn assoziiert sind, um die gewünschte Übung zu liefern.

8. Lungenübungsvorrichtung gemäß Anspruch 1, bei welcher das innere Element angepasst ist, um einer Hin- und Herbewegung in Bezug auf den Hauptkörper unterworfen werden zu können aufgrund des Ausatmens des Benutzers in das Mundstück und durch das Heben des inneren Elements, wobei die Öffnungen anfänglich durch den Hauptkörper bedeckt sind, aber bloßgelegt werden, wenn das innere Element in Bezug auf den Hauptkörper hochgehoben wird, was dazu führt, dass das innere Element in den Hauptkörper zurückfällt, um unmittelbar in Bezug auf den Hauptkörper hochgehoben zu werden, wenn der Benutzer damit fort fährt auszuatmen;
wobei der Benutzer eine schnell pulsierende Wirkung auf die Lungen erhält.

9. Beatmungsvorrichtung gemäß Anspruch 1, bei welcher das innere Element und der Hauptkörper eine Vielzahl von Einschnitten aufweisen, welche mindestens in eine erste und in eine zweite Gruppe von Einschnitten unterteil sind, und ein dazupassendes Stoppelement, wobei eine von den Gruppen der Einschnitte solche Einschnitte aufweist, welche sich am Umfang in Bezug auf das innere Element und in Bezug auf den Hauptkörper erstrecken, was eine Drehbewegung des inneren Elements in Bezug auf den Hauptkörper ermöglicht, wobei das Stoppelement sich mit den Einschnitten zusammenpaart, um das Ausmaß der Drehbewegung zu steuern, wobei die Drehbewegung eine perkussionsartige Übung ermöglicht;
die äußere Gruppe von Einschnitten solche Einschnitte aufweist, welche sich in der Längsrichtung in Bezug auf das innere Element und in Bezug auf den Hauptkörper erstrecken, was eine Hin- und Herbewegung des inneren Elements in Bezug auf den Hauptkörper ermöglicht, wobei das Stoppelement sich mit der anderen Gruppe von Einschnitten zusammenpaart, um das Ausmaß der Hin- und Herbewegung zu steuern.

10. Lungenübungsvorrichttung gemäß Anspruch 1, welche weiterhin eine Vorrichtung zum Messen eines Fortschritts aufweist.

11. Lungenübungsvorrichtung gemäß Anspruch 1, welche weiterhin eine Feder einschließt, welche das innere Element in Bezug auf den Hauptkörper vorspannt, damit das innere Element einen Widerstand aufbringen kann gegen eine Bewegung in Bezug auf den Hauptkörper, und damit das innere Element eine Hilfe erfährt für die Rückkehr in Bezug auf den Hauptkörper.

## Revendications

1. Dispositif d'exercice pulmonaire, comprenant
un corps principal;
un élément interne monté par déplacement alternatif et rotatif dans le corps principal ;
une embouchure monté sur le corps principal et en communication avec l'élément interne ;
au moins une ouverture dans l'élément interne et au moins une ouverture dans le corps principal, adaptées à être en communication sélective pour définir un passage d'air s'étendant à partir de l'embouchure à travers l'élément interne et hors du corps principal, le passage d'air pouvant être varié sélectivement lors du déplacement alternatif ou rotatif de l'élément interne par rapport a corps principal ;
le dispositif d'exercice pulmonaire pouvant ainsi fournir différents exercices pour les poumons par déplacement de l'élément interne par rapport au corps principal.

2. Dispositif d'exercice pulmonaire selon la revendication 1, dans lequel les ouvertures dans l'élément interne englobent au moins deux sorties à diamètre différent espacées longitudinalement le long de l'élément interne et adaptées à communiquer avec la au moins une ouverture dans le corps principal lors du déplacement alternatif de l'élément interne dans le corps principal ;
l'utilisateur du dispositif pouvant ainsi expirer et inspirer dans l'embouchure pour entraîner le déplacement alternatif de l'élément interne par rapport au corps principal et changer automatiquement la résistance entre l'expiration et l'inspiration.

3. Dispositif d'exercice pulmonaire selon la revendication 2, dans lequel les ouvertures dans l'élément interne englobent plusieurs sorties séparées en paires pour fournir une résistance sélective à l'inspiration et à l'expiration.

4. Dispositif d'exercice pulmonaire selon la revendication 1, dans lequel les ouvertures dans l'élément interne et le corps principal englobent des fentes pouvant être alignées sélectivement pour définir le passage d'air.

5. Dispositif d'exercice pulmonaire selon la revendication 1, dans lequel les ouvertures dans l'élément interne et le corps principal sont constituées par des fentes pouvant être alignées sélectivement par rotation de l'élément interne par rapport au corps principal, pour fermer et ouvrir ainsi le passage d'air lors du passage des fentes les unes au-dessus des autres ;
entraînant ainsi une action de percussion sur les poumons de l'utilisateur lorsque l'utilisateur inspire et expire dans l'embouchure.

6. Dispositif d'exercice pulmonaire selon la revendication 1, dans lequel les ouvertures dans l'élément interne englobent au moins deux sorties à diamètre différent espacées longitudinalement le long de l'élément interne, adaptées à communiquer avec la au moins une ouverture dans le corps principal lors du déplacement alternatif de l'élément interne dans le corps principal ; et
les ouvertures dans l'élément interne et le corps principal englobant des fentes pouvant être alignées sélectivement par rotation de l'élément interne par rapport au corps principal, pour ouvrir et fermer ainsi le passage d'air lors du passage des fentes les unes au-dessus des autres ;
un sélecteur de commande pour faire la sélection entre les sorties et les fentes ;
entraînant ainsi une action de percussion sur les poumons de l'utilisateur lorsque l'utilisateur inspire et expire dans l'embouchure lors de la sélection des fentes ou lors de la sélection des sorties, l'utilisateur pouvant entraîner le déplacement alternatif de l'élément interne par rapport au corps principal et changer automatiquement la résistance entre l'inspiration et l'expiration.

7. Dispositif d'exercice pulmonaire selon la revendication 6, dans lequel le sélecteur de commande englobe une goupille et des rainures, les rainures étant associées aux fentes ou aux sorties pour assurer l'exercice voulu.

8. Dispositif d'exercice pulmonaire selon la revendication 1, dans lequel l'élément interne est adapté à effectuer un déplacement alternatif par rapport au corps principal lorsque l'utilisateur expire dans l'embouchure, soulevant l'élément interne, les ouvertures étant initialement recouvertes par le corps principal, mais étant exposes lors du soulèvement de l'élément interne par rapport au corps principal, entraînant la retombée de l'élément interne dans le corps principal et son soulèvement immédiat par rapport au corps principal lorsque l'utilisateur continue d'expirer ;
les poumons de l'utilisateur étant ainsi soumis à un effet pulsatoire rapide.

9. Dispositif d'exercice pulmonaire selon la revendication 1, dans lequel ledit élément interne et ledit corps comportent plusieurs rainures divisées en au moins des premier et deuxième groupes de rainures, et un élément d'arrêt complémentaire, un desdits groupes de rainures comportant des rainures s'étendant circonférentiellement par rapport au dit élément interne et au dit corps principal, permettant un déplacement par rotation dudit élément interne par rapport audit corps principal, ledit élément d'arrêt étant engagé dans lesdites rainures pour contrôler l'étendue du déplacement par rotation, ledit déplacement par rotation permettant la pratique d'exercices à percussion ;
le groupe externe de rainures comportant des rainures s'étendant longitudinalement par rapport au dit élément interne et au dit corps principal, permettant un déplacement alternatif dudit élément interne par rapport au dit corps principal, ledit élément d'arrêt s'engageant dans ledit autre groupe de rainures pour contrôler l'étendue du déplacement alternatif.

10. Dispositif d'exercice pulmonaire selon la revendication 1, comprenant en outre un dispositif de mesure du progrès.

11. Dispositif d'exercice pulmonaire selon la revendication 1, comprenant en outre un ressort influençant l'élément interne par rapport au corps principal pour que l'élément interne résiste au mouvement par rapport au corps principal et pour assister le retour de l'élément interne par rapport au corps principal.
